# EUROPÄISCHE PATENTSCHRIFT

(11) **EP 2 911 600 B1**
(45) Veröffentlichungstag und Bekanntmachung des Hinweises auf die Patenterteilung: **19.04.2017**
(21) Anmeldenummer: 13783341.4
(22) Anmeldetag: 25.10.2013
(51) Int. Cl.: A61B 17/70, A61B 17/86, A61B 90/00

(54) **KNOCHENSCHRAUBE UND KNOCHENSCHRAUBENSYSTEM**
BONE SCREW AND BONE SCREW SYSTEM
VIS DE FIXATION OSSEUSE ET SYSTÈME DE VIS DE FIXATION OSSEUSE

(30) Priorität: 26.10.2012 DE 102012219630
(43) Veröffentlichungstag der Anmeldung: 02.09.2015
(73) Patentinhaber: Premiere Medical GmbH, 89155 Erbach (DE)
(72) Erfinder: VAZIFEHDAN, Farzam, 70195 Stuttgart (DE); WILLMANN, Nikolas, 89077 Ulm (DE); SCHÖNHÖFFER, Helmut, 89155 Erbach (DE)
(74) Vertreter: Kohler Schmid Möbus Patentanwälte
(86) Internationale Anmeldenummer: PCT/EP2013/072389
(87) Internationale Veröffentlichungsnummer: WO 2014/064249

(56) Entgegenhaltungen:
- US-A- 5 733 286
- US-A1- 2002 013 585
- US-A1- 2003 225 408
- US-A1- 2007 093 827
- US-A1- 2009 270 916
- US-A1- 2011 202 096
- US-A1- 2011 208 248
- US-A1- 2012 191 144
- US-A1- 2012 253 413

## Beschreibung

Die Erfindung betrifft ein Knochenschraubensystem mit einer Knochenschraube und mit einem Repositionsinstrument.

Bei der operativen Instrumentierung der Wirbelsäule werden in der klinischen Praxis Knochenschrauben mit einem am Schraubenkopf befestigten Kopfgehäuse eingesetzt. Das Kopfgehäuse weist eine Stabaufnahme für einen Fixierstab auf, der einem Aufrichten und/oder gegenseitigen Stabilisieren bzw. Führen von Wirbelkörpern dient.

Die Kopfgehäuse sind üblicher Weise als sogenannte Tulpenköpfe ausgebildet. Bei den sogenannten monoaxialen Knochenschrauben sind die Kopfgehäuse am Schraubenkopf drehfest oder einachsig verdrehbar angeordnet. Bei den sogenannten polyaxialen Knochenschrauben können die Kopfgehäuse gegenüber der Längsachse des Gewindeschafts ein- oder mehrachsig ausgelenkt werden.

Bei einer langstreckigen offenen Instrumentierung der Wirbelsäule (="Mehretageninstrumentierung"), wie diese beispielsweise bei Operationen zur Korrektur von Wirbelsäulendeformitäten, insbesondere einer Skoliose, erforderlich ist, muss der eingesetzte Fixierstab in der Stabaufnahmen einer Vielzahl von Knochenschrauben positioniert und fixiert werden. Die in der Praxis eingesetzten Fixierstäbe sind in der Regel äußerst biegesteif und werden bevorzugt außerhalb des Körpers in eine gewünschte Form vorgebogen. Sobald der Fixierstab in der Stabaufnahme einer anderen Knochenschraube gehalten angeordnet ist, kann dieser deshalb oftmals nur unter hohem Aufwand, beispielsweise mittels eines sogenannten "rod-benders", nachträglich in Form gebogen bzw. in die Stabaufnahme herkömmlicher Lang-Kopfgehäuse eingebracht werden. Dazu muss der Fixierstab in der Regel über einen der beiden Wandschenkel des Kopfgehäuses (hinweg-)gehebelt werden. In der Praxis kommt es dadurch häufig zu einer übermäßigen Gewebstraumatisierung, die u.a. Wundinfektion Vorschub leisten kann.

US 2002/013585 A1 offenbart beispielsweise ein Knochenschraubensystem mit einem Repositionsinstrument und mit einer Knochenschraube für Implantate zur Korrektur und Stabilisierung der Wirbelsäule. Die Knochenschraube weist einen Gewindeschaft mit einem Schraubenkopf mit einem Antriebsprofil auf. Ein Kopfgehäuse ist über einen Kopplungsabschnitt mit dem Schraubenkopf verbunden. Das Kopfgehäuse weist eine Stabaufnahme für einen Fixierstab auf, die von einem ersten und einem zweiten Wandschenkel seitlich begrenzt ist, die sich in Richtung einer Gehäuselängsachse vom Kopplungsabschnitt wegerstrecken. Die beiden Wandschenkel weisen jeweils ein Innengewindesegment für eine Fixierschraube auf, mittels derer der Fixierstab in der Stabaufnahme festlegbar ist. Der zweite Wandschenkel überragt den ersten Wandschenkel in axialer Richtung. Der erste Wandschenkel weist an seinem freien Randabschnitt ein erstes Kopplungsmittel zum Ankoppeln des Repositionsinstruments auf. Der zweite Wandschenkel des Kopfgehäuses ist mit einer Sollbruchstelle versehen, die axial auf Höhe oder im Wesentlichen auf Höhe des freien Endes des ersten Wandschenkels angeordnet ist.

US 2003/225408 A1 offenbart ein weiteres Knochenschraubensystem mit einer Knochenschraube und mit einem Repositionsinstrument zum Einbringen und Fixieren eines Fixierstabs in der Stabaufnahme des Kopfgehäuses der Knochenschraube. Das Repositionsinstrument weist eine Führungshülse mit einer längsaxialen Durchgangsbohrung und mit einem Wandprofil auf, das sich in axialer Richtung von der Führungshülse wegerstreckt. Das Wandprofil weist an seinem freien Ende ein zweites Kopplungsmittel auf, das zu dem ersten Kopplungsmittel desersten Wandschenkels des Kopfgehäuses der Knochenschraube komplementär ausgebildet ist. Ein Arretiermittel dient einem Arretieren der Führungshülse an den beiden Wandschenkeln des Kopfgehäuses. Ein Druckglied zum Absenken des Fixierstabs ist in der Durchgangsbohrung der Führungshülse axial verstellbar angeordnet. Ein Drehwerkzeug ist an der Führungshülse relativ zu dieser drehbar gelagert, wobei das Drehwerkzeug mit dem Druckglied derart gekoppelt ist, dass das Druckglied durch ein Verdrehen des Drehwerkzeugs relativ zur Führungshülse axial verstellbar ist.

Weitere Knochenschraubensysteme mit einer Knochenschraube und einem Repositionsinstrument zum Einbringen und Fixieren eines Fixierstabs in der Stabaufnahme des Kopfgehäuses der Knochenschraube sind aus der US 2012/0191144 A1, der US 2011/0202096 A1 und der US 2012/0253413 A1 bekannt. US 2009/0270916 A1 offenbart eine Knochenschraube mit unterschiedlich langen Wandschenkeln. Weitere Knochenschrauben sind in der US 2011/0208248 A1, der US 2007/0093827 A1 und der US 5,733,286 A bekannt.

Aufgabe der Erfindung ist es, ein Knochenschraubensystem mit einer Knochenschraube und mit einem Repositionsinstrument anzugeben, die gegenüber den bekannten Knochenschraubensystemen bei vereinfachter Handhabung eine geringere Gewebetraumatisierung selbst bei minimalinvasiven operativen Zugängen ermöglichen.

Die das Knochenschraubensystem betreffende Aufgabe wird durch ein Knochenschraubensystem mit den in Patentanspruch 1 angegebenen Merkmalen gelöst.

Weiterbildungen der Erfindung sind Gegenstand von Unteransprüchen.

Das erfindungsgemäße Knochenschraubensystem erlaubt nicht zuletzt durch die unterschiedlich langen Wandschenkel der Stabaufnahme des Kopfgehäuses der Knochenschraube ein kraftschonendes, weniger traumatisches und deutlich vereinfachtes Einbringen und Fixieren des Fixierstabs in der Stabaufnahme. Dies insbesondere dann, wenn der Fixierstab bereits an/in anderen Knochenschrauben festgesetzt ist. Erforderliche Operationszeiten können dadurch insgesamt verkürzt und zeitraubende sowie häufig traumatisierende Revisionen an der Instrumentierung vermieden werden. Der längere zweite Wandschenkel des Köpfgehäuses kann nach dem Festlegen bzw. Fixieren des Fixierstabs im Kopfgehäuse mit einfachen Mitteln, beispielsweise einer Zange oder dergl. im Bereich der Sollbruchstelle abgebrochen und so auf ein erforderliches Mindestmaß gekürzt werden. Durch die zum Arretieren des Repositionsinstruments am Kopfgehäuse der Knochenschraube erforderliche Schwenkbewegung des Instruments gegenüber dem Kopfgehäuse kann ein Fixierstab ohne großen Kraftaufwand mit dem Instrument in Richtung auf die Gehäuseachse des Kopfgehäuses geführt (gehebelt) und zwischen dem Wandprofil der Führungshülse des Repositionsinstruments und dem (längeren) zweiten Wandschenkel des Kopfgehäuses gefangen werden. Der Fixierstab kann mittels des Repositionswerkzeugs in der Stabaufnahme des Kopfgehäuses auf einfache und kontrollierte Weise abgesenkt werden. Die Fixierschraube kann nachfolgend in die Gewindesegmente der Stabaufnahme eingeschraubt wird, um den Fixierstab in der Stabaufnahme der Knochenschraube festzusetzen. Durch die Entkopplung des Reponierens bzw. Absenken des Fixierstabs in der Stabaufnahme und des Einschraubens der Fixierschraube mit Hilfe eines Schraubendrehers oder dergl. wird eine besonders einfache, zügige und zugleich sichere Handhabung des Knochenschraubensystems ermöglicht.

Das Arretiermittel des Repositionswerkzeugs ist im konstruktiv einfachsten Fall als eine Arretierhülse ausgebildet, die die Führungshülse umgreift und die auf der Arretierhülse in axialer Richtung zwischen einer Freigabestellung und einer Arretierstellung relativ zur Führungshülse verstellbar ist. Die Arretierhülse ist dazu ausgebildet, in der ihrer Arretierstellung den ersten Wandschenkel und den zweiten Wandschenkel des Kopfgehäuses der Knochenschraube außenseitig zu über- bzw. zu umgreifen.

Die Arretierhülse kann erfindungsgemäß einen Arretierarm aufweisen, der sich von der Arretierhülse in axialer Richtung wegerstreckt und der in Arretierstellung der Arretierhülse in eine Arretierausnehmung des ersten Wandschenkels der Knochenschraube eingreift. Dadurch kann einem unerwünschten Abrutschen des Arretierarms von dem ersten Wandschenkel der Stabaufnahme der Knochenschraube zuverlässig entgegengewirkt werden.

Die Arretierhülse kann zusätzlich einen Arretierfortsatz aufweisen, der an einem dem Arretierarm in radialer Richtung gegenüberliegen Hülsenabschnitt der Arretierhülse angeordnet ist. Der Arretierfortsatz erstreckt sich von der Arretierhülse in axialer Richtung weg und ist zum Arretierarm der Arretierhülse vorzugsweise parallel verlaufen angeordnet. Der Arretierarm ist dazu ausgebildet, das zweite Wandprofil der Knochenschraube in der Arretierstellung der Arretierhülse außenseitig zu übergreifen. Der Arretierfortsatz kann dabei, beispielsweise einen Arretierstift aufweisen, der dazu ausgebildet ist, in eine Arretierausnehmung des zweiten Wandschenkels des Kopfgehäuses der Knochenschraube eingreifen. Insgesamt kann dadurch kann eine mechanisch hoch belastbare Verkopplung des Repositionsinstruments mit dem Kopfgehäuse realisiert werden. Einem unerwünschten Abrutschen des Repositionsinstruments aus seiner mit dem Kopfgehäuse arretierten Funktionsstellung kann zuverlässig entgegengewirkt werden. Dies ist für die Bediensicherheit des Knochenschraubensystems vorteilhaft.

Die Arretierhülse kann erfindungsgemäß an der Führungshülse drehfest angeordnet sein. Dies kann beispielsweise durch einen Sicherungsstift realisiert sein, der in ein Langloch bzw. eine Führungsnut der Arretier- oder Führungshülse eingreift.

Zur Armierung bzw. verbesserten Führung des Arretierarms hat es sich als vorteilhaft erwiesen, wenn die Führungshülse eine Führungsausnehmung aufweist, in der der Arretierarm der Arretierhülse verschiebbar geführt ist. Die Führungsausnehmung ist bevorzugt durch einen seitlichen Wandfortsatz der Führungshülse gebildet. Insgesamt kann dadurch einem unerwünschten seitlichen Abrutschen des Arretierarms von dem Kopfgehäuse bzw. einer Überbelastung des Arretierarms in seiner Arretierstellung relativ zum Kopfgehäuse entgegengewirkt werden.

Die Handhabung des Repositionsinstruments kann dadurch nochmals weiter vereinfacht und sicherer gemacht werden, dass das Arretiermittel an der Führungshülse in Arretierstellung vorgespannt gehalten ist. Die Arretierhülse kann dazu insbesondere mittels einer vorgespannten Druckfeder an der Führungshülse abgestützt sein.

Das Drehwerkzeug bzw. der Drehknebel des Repositionsinstruments kann im Hinblick auf eine einfache Montage sowie eine vereinfachte Reinigung des Repositionswerkzeugs an der Führungshülse axial lagefixiert sein. Das Drehwerkzeug steht in diesem Fall mit dem Druckglied vorzugsweise in einem lösbaren Gewindeeingriff. D.h. das Druckglied ist vorzugsweise in das Drehwerkzeug eingeschraubt.

Das Drehwerkzeug und das Druckglied weisen im konstruktiv einfachsten Fall jeweils einen axial verlaufenden Werkzeugdurchgang für einen Schraubendreher zum Einschrauben der Fixierschraube in das Kopfgehäuse der Pedikelschraube auf.

Für ein sicheres Halten der Fixierschraube kann das Druckglied vorzugsweise mit einer Halteeinrichtung für die Fixierschraube versehen sein. Das Druckglied kann insbesondere ein Federelement, bevorzugt einen Federring, zum Halten der Fixierschraube aufweisen.

Im Hinblick auf eine besonders einfache und sichere Handhabung des Positionierinstruments hat es sich als vorteilhaft erwiesen, wenn die Führungshülse eine Anschlagfläche für den zweiten Wandabschnitt des Kopfgehäuses aufweist. Die Anschlagfläche kann insbesondere durch eine randseitige Schulter der Führungshülse gebildet sein. Dadurch kann die Funktionsstellung des Repositionswerkzeugs am Kopfgehäuse der Knochenschraube zügiger und sicher aufgefunden werden. Die Funktionsstellung des Repositionsinstruments ist diejenige Ankoppelstellung des Repositionsinstruments an dem Kopfgehäuse der Knochenschraube, in der das Repositionsinstrument an dem Kopfgehäuse mittels des Arretiermittels bzw. der Arretierhülse arretierbar ist.

Nach der Erfindung kann die Sollbruchstelle des zweiten Wandschenkels des Kopfgehäuses der Knochenschraube zumindest eine umfangsseitige Kerbe des zweiten Wandschenkel umfassen. Für ein besonders atraumatisches Kürzen des zweiten Wandschenkels hat es sich als vorteilhaft erwiesen, wenn der Wandschenkel innen- und außenseitig mit einer solchen Kerbe versehen ist. Die Sollbruchstelle kann auch durch eine anders geartete Materialschwächung des zweiten Wandschenkels erzeugt sein.

Das Innengewindesegment des ersten Wandschenkels erstreckt sich in axialer Richtung vorteilhaft bis zum freien Ende des ersten Wandschenkels und das Innengewindesegment des zweiten Wandschenkels erstreckt sich vorteilhaft bis zur oder nahe bis zur Sollbruchstelle des zweiten Wandschenkels. Dadurch kann die Fixierschraube zuverlässig und sicher in das Kopfgehäuse eingeschraubt werden. Einer unerwünschten Beschädigung des Innengewindesegments des zweiten Wandschenkels durch das Kürzen des zweiten Wandschenkels kann darüber hinaus zuverlässig entgegengewirkt werden.

Nach einer bevorzugten Weiterbildung der Erfindung ist das Kopfgehäuse am Schraubenkopf relativ zur Längsachse des Gewindeschafts der Knochenschraube auslenkbar angeordnet und/oder um seine Gehäuselängsachse frei drehbar am Schraubenkopf angeordnet. Dadurch kann das Kopfgehäuse der Knochenschraube vereinfacht relativ zu dem darin festzusetzenden Fixierstab bzw. zu einem nachstehend beschriebenen Repositionswerkzeug ausgerichtet werden.

Das Antriebsprofil der Knochenschraube kann erfindungsgemäß von einem Einsatzteil gebildet sein, das in einer Lageraufnahme des Schraubenkopfs angeordnet ist, wobei das Einsatzteil mit dem Schraubenkopf in Richtung um die Längsachse des Gewindeschafts der Knochenschraube drehfest verkoppelt ist und wobei das Einsatzteil am Kopfgehäuse geführt gehalten und gemeinsam mit diesem gegenüber der Längsachse des Gewindeschafts auslenkbar ist. Dadurch kann das Antriebsprofil der Knochenschraube unabhängig von einer Auslenkung (einem Verkippen/Abklappen) des Kopfgehäuses gegenüber der Längsachse des Gewindeschafts der Knochenschraube zu jedem Zeitpunkt mit der Gehäuseachse des Kopfgehäuses ausgerichtet ist bzw. mit dieser fluchtet. Ein Drehwerkzeug kann dadurch einfacher, zügiger und sicherer an das Antriebsprofil der Knochenschraube herangeführt und mit diesem drehschlüssig verkoppelt werden. Ein zeitraubendes, traumatisierendes und die Hardware ggf. beschädigendes Aufsuchen eines zum Ankoppeln an das Antriebsprofil geeigneten Ansetzwinkels des Drehwerkzeugs am Schraubenkopf der Knochenschraube entfällt. Einer übermäßigen Traumatisierung des umgebenden Weichteilgewebes kann dadurch selbst bei einem minimalinvasiven operativen Zugangsweg zuverlässig entgegengewirkt werden. Die Knochenschraube kann als Wirbelkörperschraube zum Einschrauben in den Wirbelkörper eines Wirbels bzw. als sogenannte Pedikelschraube ausgeführt sein.

Das Einsatzteil kann im konstruktiv einfachsten Fall einen (ersten) Lagerzapfen, bevorzugt zwei (erste) Lagerzapfen, aufweisen, der/die sich jeweils in radialer Richtung vom Einsatzteil wegerstreckt bzw. wegerstrecken. Der bzw. die Lagerzapfen greift/greifen dabei bevorzugt jeweils in eine Seitenwandausnehmung der Lageraufnahme des Schraubenkopfs ein. Die Seitenwandausnehmungen können jeweils als nutartige Vertiefungen der Seitenwand oder aber als Wanddurchbrüche ausgebildet sein. Das Einsatzteil kann dadurch nicht nur um eine durch die Lagerzapfen definierte erste Dreh- bzw. Kippachse gegenüber dem Schraubenkopf verkippt werden, sondern auch um eine zu dieser orthogonal ausgerichtete zweite Dreh- bzw. Kippachse in der/denn Seitenwandausnehmung(en) des Schraubenkopfs gegenüber dem Schraubenkopf bzw. der Längsachse des Gewindeschafts der Knochenschraube verkippt werden. Es versteht sich, dass das erfindungsgemäße Einsatzteil mit dem Antriebsprofil auch für den Einsatz bei mehrstückig ausgeführten monoaxialen Knochenschrauben, insbesondere mit einem abgewinkelten Kopfgehäuse, geeignet ist.

Die Seitenwandausnehmung/-en der Lageraufnahme sind erfindungsgemäß vorzugsweise zum freien Ende des Schraubenkopfs hin offen. Dadurch kann das Einsatzteil vereinfacht am Schraubenkopf montiert werden.

Nach einer besonders bevorzugten Weiterbildung der Erfindung ist das Einsatzteil an einem in der Lageraufnahme verschwenkbar angeordneten Kardanring gelagert. Der erste bzw. die ersten Lagerzapfen des Einsatzteils greifen dabei bevorzugt in jeweils eine Seitenwandausnehmung des Kardanrings ein. Dadurch kann insgesamt ein nochmals vergrößerter Bewegungsumfang des Einsatzteils bzw. des Kopfgehäuses relativ zur Längsachse des Gewindeschafts der Knochenschraube realisiert werden. Darüber hinaus ergibt sich dadurch eine besonders leichtgängige Lagerung des Einsatzteils am Schraubenkopf. Dies ist für ein in der klinischen Praxis oftmals wesentliches feinfühliges Ausrichten des Kopfgehäuses von Vorteil. Der Kardanring greift vorzugsweise mit zweiten Lagerzapfen in Seitenwandausnehmungen des Schraubenkopfs ein, die zum (kopfseitigen) freien Ende der Knochenschraube hin bevorzugt offen ausgebildet sind. Die Seitenwandausnehmungen können dabei als nutartige Einbuchtungen bzw. Vertiefungen der Seitenwand oder aber als Wanddurchbrüche ausgebildet sein.

Das Einsatzteil kann nach der Erfindung zumindest abschnittsweise eine sphärische Außenkontur bzw. Außenseite und die Lageraufnahme des Schraubenkopfs zumindest abschnittsweise eine, insbesondere dazu korrespondierend ausgebildete, gewölbte Seitenwand aufweisen. Dies ist für die mechanische Belastbarkeit der Einsatzteil-Schraubenkopfkopplung von Vorteil. Darüber hinaus kann die Lageraufnahme des Schraubenkopfs beim Festsetzen eines Fixierstabs in der Stabaufnahme durch das Einsatzteil in radialer Richtung aufgeweitet und dadurch mit dem Kopfgehäuses verklemmt werden. Im Hinblick auf eine optimierte Klemmwirkung hat es sich als vorteilhaft erwiesen, wenn die Außenkontur bzw. Außenseite des Einsatzteils vielfach facettiert ausgeführt ist. Der Schraubenkopf kann nach der Erfindung darüber hinaus mehrfach geschlitzt sein, um ein gleichmäßiges Aufdehnen (Aufweiten) des Schraubenkopfes zu ermöglichen.

Das Einsatzteil kann erfindungsgemäß insbesondere mit einem ringbundförmigen Führungsabschnitt am Kopfgehäuse innenseitig anliegen. Dadurch kann das Kopfgehäuse überdies um seine Gehäuselangsachse gegenüber dem Schraubenkopf frei verdreht werden. Unter fertigungstechnischen Gesichtspunkten ist das Einsatzteil vorteilhaft mittels des Kopfgehäuses in der Lageraufnahme des Schraubenkopfs axial lagegesichert.

Eine besonders einfache Montage eines Kopfgehäuses an einem Schraubenkopf einer Knochenschraube kann erfindungsgemäß dadurch realisiert werden, dass das Kopfgehäuse über einen Federring mit dem Schraubenkopf der Knochenschraube unlösbar verrastet ist. Diese unlösbare Rastverbindung des Kopfgehäuses mit dem Schraubenkopf kann nicht zerstörungsfrei gelöst werden.

Der Federring ist vorzugsweise geschlitzt ausgeführt und kann nach der Erfindung in radialer Richtung innen- oder außenspannend ausgeführt sein. Auf zusätzliche Sicherungselemente kann dadurch verzichtet werden.

Im Falle eines innenspannenden Federrings weist das Kopfgehäuse erfindungsgemäß vorzugsweise eine Schraubenkopfaufnahme auf, deren Seitenwand mit einer Ringnut versehen ist. Die Ringnut ermöglicht, dass der Federring bei einem axialen Aufstecken des Kopfgehäuses auf den Schraubenkopf in radialer Richtung ausweichen kann, um eine Passage (insbesondere der Äquatorialebene) des Schraubenkopfs durch den Federring hindurch zu ermöglichen. Innerhalb der Ringnut ist vorteilhaft ein Vorsprung für eine radiale Begrenzung einer Aufweitung des Federrings angeordnet. Der Vorsprung ist dabei vorzugsweise in einem unteren (gewindeschaftseitigen) Bereich der Nut angeordnet. Der Vorsprung kann insbesondere ringförmig umlaufend ausgebildet sein.

Zur axialen Lagesicherung des Federrings in seiner Sperr- bzw. Raststellung kann das Kopfgehäuse erfindungsgemäß ein Sicherungsmittel, beispielsweise einen Sicherungsstift aufweisen, der sich durch die Wand des Kopfgehäuses hindurcherstreckt und der in die Ringnut des Kopfgehäuses hineinragt.

Ein unerwünschtes axiales Entfernen bzw. Herausfallen des Federrings aus der Schraubenkopfaufnahme des Kopfgehäuses kann erfindungsgemäß dadurch unterbunden werden, dass die Schraubenkopfaufnahme mit einem axialen Anschlag für den Federring versehen ist. Der Anschlag kann insbesondere durch einen Seitenwandvorsprung, beispielsweise einen Ringbund, eine Nutflanke oder dergl., gebildet sein.

Nach einer bevorzugten Weiterbildung der Erfindung weist das Kopfgehäuse einen Kopplungsabschnitt mit einer Schraubenkopfaufnahme auf, die eine gewölbte Innenwand aufweist. Die Schraubenkopfaufnahme kann auch konisch ausgeformt sein. Dadurch kann eine (polyaxiale) Anlenkung des Kopfgehäuses an einem sphärischen Schraubenkopf einer Knochenschraube auf einfache Weise realisiert werden. D. h., das Kopfgehäuse kann dadurch am Schraubenkopf relativ zur Längsachse des Gewindeschafts der Knochenschraube ein- oder mehrachsig verkippbar angeordnet werden.

Weitere Vorteile und vorteilhafte Ausgestaltungen des Gegenstands der Erfindung ergeben sich aus der Beschreibung, den Ansprüchen und der Zeichnung. Ebenso können die vorstehend genannten und die noch weiter aufgeführten Merkmale je für sich oder zu mehreren in beliebigen Kombinationen Verwendung finden.

Nachfolgend wird die Erfindung anhand eines in der Zeichnung wiedergegebenen Ausführungsbeispiels näher erläutert. Die gezeigte und beschriebene Ausführungsform ist nicht als abschließende Aufzählung zu verstehen, sondern hat vielmehr beispielhaften Charakter für die Schilderung der Erfindung.

In der Zeichnung zeigen:
- Fig. 1: eine Knochenschraube mit einem am Schraubenkopf der Knochenschraube polyaxial angelenkten Kopfgehäuse, das eine Stabaufnahme für einen Fixierstab aufweist, wobei die Stabaufnahme von zwei Wandschenkeln mit unterschiedlicher Länge begrenzt ist und wobei einer der beiden Schenkel mit einer Sollbruchstelle versehen ist;
- Fig. 2: die Knochenschraube aus Fig. 1, in einer anderen Seitenansicht;
- Fig. 3: die Knochenschraube aus Fig. 1, in einer perspektivischen Ansicht;
- Fig. 4: die Knochenschraube aus Fig. 1, in einem Längsschnitt;
- Fig. 5: die Knochenschraube aus Fig. 1, in einer ausschnittsweise explodierten Darstellung ihrer Teile;
- Fig. 6: eine Knochenschraube gemäß Fig. 1, bei der das Antriebsprofil der Knochenschraube von einem Einsatzteil gebildet ist, das am Schraubenkopf der Knochenschraube kardanisch gelagert ist;
- Fig. 7: ein Knochenschraubensystem mit einer Knochenschraube gemäß Fig. 1, mit einem Repositionswerkzeug und mit einem Fixierstab beim Ankoppeln des Repositionsinstruments an die Knochenschraube, in Seitenansicht;
- Fig. 8: das Knochenschraubensystem gemäß Fig. 7 in einer anderen Seitenansicht;
- Fig. 9: ein Knochenschraubensystem, das zu dem Knochensystem gemäß Fig. 7 ähnlich ist, in einem Längsschnitt;
- Fig. 10: eine ausschnittsweise detailvergrößerte Ansicht des Knochenschraubensystems aus Fig. 7;
- Fig. 11: das Knochenschraubensystem gemäß Fig. 9 in einer anderen Betriebsstellung des Repositionswerkzeugs an der Knochenschraube, im Längsschnitt;
- Fig. 12: das Knochenschraubensystem gemäß Fig.11 in einer ausschnittsweise vergrößerten perspektivischen Ansicht;
- Fig. 13: das Knochenschraubensystem gemäß Fig. 9 mit einem in seiner Funktionsstellung an der Knochenschraube arretierten Repositionswerkzeug, im Längsschnitt;
- Fig. 14: das Knochenschraubensystem gemäß Fig. 14 in einer perspektivischen Ansicht;
- Fig. 15: das Knochenschraubensystem gemäß Fig. 15 in einer ausschnittsweise detailvergrößerten perspektivischen Ansicht;
- Fig. 16: das Knochenschraubensystem gemäß Fig. 15 in einer anderen ausschnittsweise detailvergrößerten perspektivischen Ansicht;
- Fig. 17: das Knochenschraubensystem gemäß Fig. 15 in einer anderen Betriebszustand, in dem der Fixierstab in der Stabaufnahme vollständig abgesenkt ist und in axialer Richtung an der Knochenschraube anliegt, in einer perspektivischen Ansicht;
- Fig. 18: einen in Fig. 17 mit x bezeichneten Bereich des Knochenschraubensystems in einer detailvergrößerten perspektivischen Ansicht;
- Fig. 19: das Knochenschraubensystem gemäß Fig. 17 in einem vergrößerten Detailausschnitt und im Längsschnitt,
- Fig. 20: das Knochenschraubensystem gemäß Fig. 17 mit einem in das Repositionsinstrument eingeführten Schraubendreher zum Fixieren des Fixierstabs mittels einer Fixierschraube, im Längsschnitt; und
- Fig. 21: einen in Fig. 20 mit x gekennzeichneten Ausschnitt des Knochenschraubensystems in einer vergrößerten Schnittdarstellung.

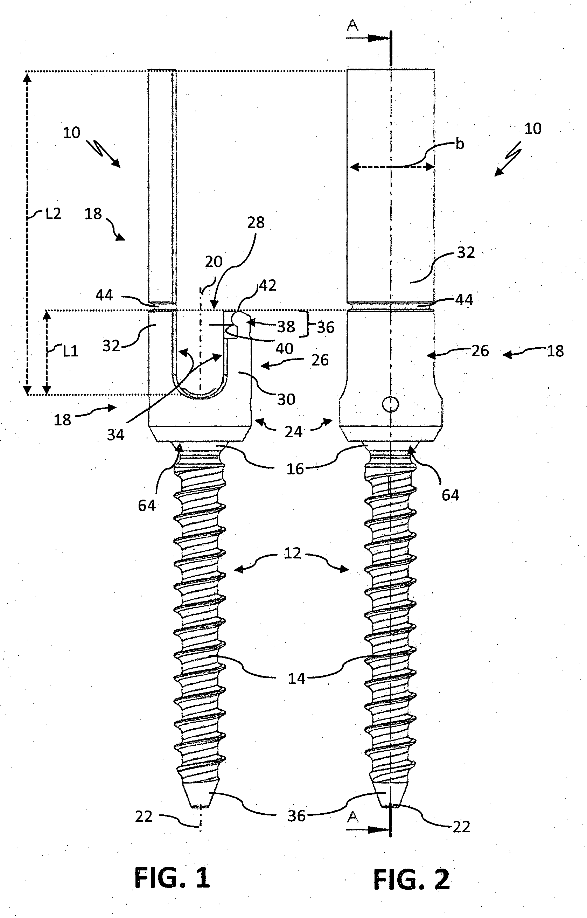

In den **Figuren 1** und **2** ist eine Knochenschraube **10** für Implantate zur Korrektur und Stabilisierung der Wirbelsäule in Seitenansicht gezeigt. Die Knochenschraube 10 ist speziell auf den Gebrauch bei langstreckigen offenen Instrumentierung der Wirbelsäule, insbesondere zur Korrektur von Wirbelsäulendeformitäten, ausgerichtet und kann insbesondere auch bei minimalchirurgische Operationstechniken eingesetzt werden. Die Knochenschraube 10 umfasst einen Gewindeschaft **12** mit einem Gewinde **14,** einem Schraubenkopf **16** sowie ein am Schraubenkopf 16 polyaxial angelenktes Tulpen- oder Kopfgehäuse **18**. D. h. das Kopfgehäuse 18 kann mit seiner Gehäuselängsachse **20** gegenüber der Längsachse **22** des Gewindeschafts 12 der Knochenschraube 12 (mehrachsig) frei ausgelenkt und vorliegend zusätzlich gegenüber dem Schraubenkopf 16 um die Längsachse 22 des Gewindeschafts 12 der Knochenschraube 10 frei verdreht werden.

Das Kopfgehäuse 18 ist vorliegend gegenüber dem Schraubenkopf 16 in seiner Neutralstellung angeordnet. In Neutralstellung fallen die Gehäuselängsachse 20 und die Längsachse 22 der Knochenschraube 10 zusammen, d.h. das Kopfgehäuse 18 ist gegenüber der Längsachse 22 nicht abgeklappt.

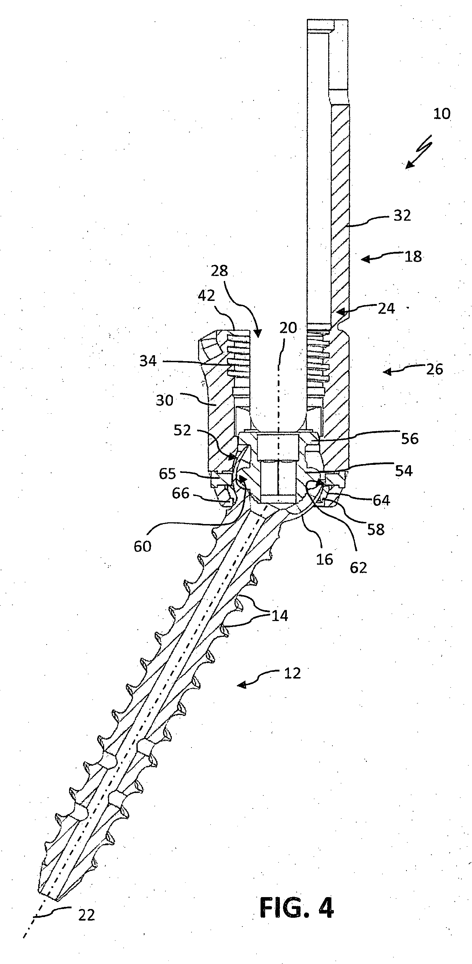

Das Kopfgehäuse 18 weist einen ringförmigen Kopplungsabschnitt **24** und einen Aufnahmeabschnitt **26** auf. Der Kopplungsabschnitt 24 dient einem Verkoppeln des Kopfgehäuses 18 mit dem Schraubenkopf 16 der Knochenschraube 12. Der Kopplungsabschnitt 26 weist eine in Fig. 4 näher wiedergegebene Schraubenkopfaufnahme für den Schraubenkopf 16 der Knochenschraube 10 auf.

Der Aufnahmeabschnitt 26 des Kopfgehäuses 18 weist eine Stabaufnahme **28** für einen nicht näher wiedergegebenen Fixierstab auf, wie dieser in der Praxis beispielsweise zur interpedikulären Instrumentierung der Wirbelsäule (Spondylodese) eingesetzt wird.

Die Stabaufnahme 28 des Kopfgehäuses 18 ist durch einen ersten und einen zweiten Wandschenkel **30**, **32** seitlich, d.h. in radialer Richtung zur Gehäuselängsachse 20, begrenzt. Die beiden Wandschenkel 30, 32 sind voneinander beabstandet angeordnet und erstrecken sich parallel zur Gehäuselängsachse 20 vom Kopplungsabschnitt 24 weg. Die Wandschenkel 30, 32 und weisen jeweils ein Innengewindesegment **34** auf, in die eine nicht näher wiedergegebene Fixierschraube eingeschraubt werden kann, um den Fixierstab in der Stabaufnahme des Kopfgehäuses 18 festzusetzen. Die Innengewindesegmente 34 können selbsthemmend ausgebildet sein.

Der kürzere erste Wandschenkel 30 weist an seinem freien Randabschnitt **36** zwei Kopplungsmittel **38** zum Ankoppeln eines nachstehend erläuterten Repositionsinstruments auf. Die beiden Kopplungsmittel 38 sind beispielhaft jeweils als zungenartige Wandfortsätze ausgebildet, die in Umfangsrichtung zu jeweils einer Seite des kürzeren ersten Wandschenkels 30 angeordnet sind. Die beiden Kopplungsmittel 38 weisen jeweils eine dem Kopplungsabschnitt 24 des Kopfgehäuses 18 zuweisende, hier beispielhaft konvex ausgeführte, Hinterschneidung **40** auf.

Der längere zweite Wandschenkel 32 steht in Richtung der Gehäuselängsachse 20, d.h. in axialer Richtung, über den ersten Wandschenkel hervor. Eine Länge **L2** des zweiten Wandschenkels 32 beträgt ungefähr das 4 fache der Länge **L1** des kürzeren ersten Wandschenkels 30. Die Länge L2 des zweiten Wandschenkels 32 kann grundsätzlich das zwei- bis sechsfache der Länge L1 des ersten Wandschenkels 30 betragen.

Der zweite Wandschenkel 32 weist ungefähr auf axialer Höhe des freien Rands bzw. freien Endes **42** des ersten Wandschenkels eine Sollbruchstelle **44** auf. Die Sollbruchstelle 44 ist durch eine außenseitige Kerbe des zweiten Wandschenkels 32 gebildet. Die Kerbe erstreckt sich in Umfangsrichtung über die gesamte Breite **b** des zweiten Wandschenkels 32. Die Sollbruchstelle 44 kann auch durch eine innenseitige und eine außenseitige Kerbe oder eine anders geartete Materialschwächung des Materials des zweiten Wandschenkels 32 gebildet sein.

Das Innengewindesegment 34 des ersten Wandschenkels 30 erstreckt sich in axialer Richtung bis zum freien Ende 42 des ersten Wandschenkels 30. Das Innengewindesegment 34 des zweiten Wandschenkels 32 erstreckt sich bis zur Sollbruchstelle 44.

Der Gewindeschaft 12 der Knochenschraube 10 kann eine oder mehrere seitliche Ausführungsöffnungen (nicht gezeigt) aufweisen, die das Einbringen von Fremdmaterial in ein den Gewindeschaft 12 umgebendes Knochengewebe erlauben.

Die Knochenschraube kann grundsätzlich auch als sogenannte monoaxiale Knochenschraube ausgebildet sein.

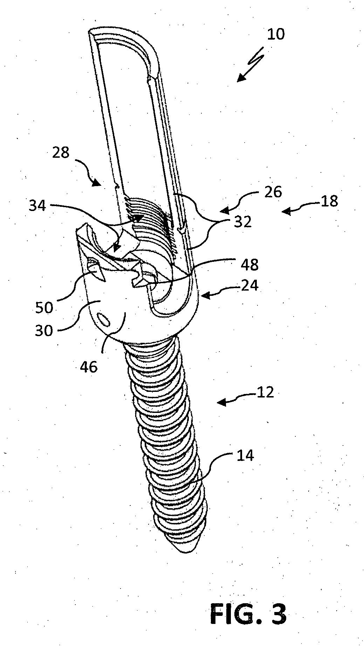

**Fig. 3** zeigt die Knochenschraube 10 in einer perspektivischen Ansicht. Das Kopfgehäuse 18 weist eine im Wesentlichen kreiszylindrische Außenmantelfläche **46** auf. Ein Antriebsprofil **48** der Knochenschraube 10 dient dem Angriff eines nicht näher wiedergegebenen Drehwerkzeugs, mit dessen Hilfe die Knochenschraube 10 in den Pedikel oder den Wirbelkörper eines Wirbels (nicht gezeigt) eingedreht bzw. aus diesem wieder herausgedreht werden kann. Das Antriebsprofil 48 ist vorliegend als Innensechskant ausgeführt, kann aber auch andersartig profiliert sein.

Der erste Wandschenkel 30 der Stabaufnahme 28 des Kopfgehäuses weist außenseitig eine mit bezeichnete erste Arretierausnehmung **50** für das nachstehend erläuterte Repositionsinstrument auf. Die erste Arretierausnehmung 50 ist nutförmig ausgebildet und zum freien Ende 42 des ersten Wandschenkel 30 hin offen.

**Fig. 4** zeigt die Knochenschraube 10 im Längsschnitt. Das Kopfgehäuse 18 ist gegenüber der Längsachse 22 der Knochenschraube 10 abgewinkelt (abgeklappt) angeordnet.

Der Schraubenkopf 16 ist teilweise sphärisch ausgebildet. In einer gewölbten Lageraufnahme **52** des Schraubenkopfs 16 ist ein Einsatzteil **54** angeordnet, das das Antriebsprofil 48 der Knochenschraube 10 bildet. Das Einsatzteil 54 ist mit Schraubenkopf 16 der Knochenschraube 10 drehfest verkoppelt.

Das Einsatzteil 54 weist einen zu der Lageraufnahme 52 im Wesentlichen korrespondierend ausgeformte kugelförmige Außenseite auf und liegt an einer Seitenwand der Lageraufnahme 52 zumindest abschnittsweise im Gleitspiel-Formschluss an.

Das Einsatzteil 54 ragt in axialer Richtung aus der Lageraufnahme 52 hervor und erstreckt sich in das Kopfgehäuse 18 hinein. Das Einsatzteil 54 ist mit einem ringbundförmigen Führungsabschnitt 56 in radialer Richtung an dem Kopfgehäuse 18 geführt gehalten.

Das Kopfgehäuse 18 ist über einen Federring **58** am Schraubenkopf 16 der Knochenschraube 10 axial lagegesichert und unlösbar mit dem Schraubenkopf verrastet. Der Federring 58 umgreift den Schraubenkopf 16 und kann geschlitzt ausgeführt sein. Das Kopfgehäuse 18 ist im Bereich der Schraubenkopfaufnahme **60** des Kopplungsabschnitts 24 innenseitig mit einer Ringnut **62** versehen, die konzentrisch zur Gehäuselängsachse 20 angeordnet ist. Die Ringnut 62 kann auch, insbesondere bei einem abgewinkelt ausgeführten Kopfgehäuse 18 gegenüber der Längsachse 22 verkippt angeordnet sein.

Der Federring 58 kann bei einem axialen Einführen des Schraubenkopfs 16 in die Schraubenkopfaufnahme 60 des Kopplungsabschnitts 24 durch den Schraubenkopf 16 (auf-)geweitet und dabei in radialer Richtung in die Ringnut 62 hinein ausweichen. Nach einem Passieren der Äquatorialebene des Schraubenkopfs 16 rückt der Federring 58 selbsttätig aus der Ringnut 62 aus. Ein innerhalb der Ringnut 62 angeordneter Vorsprung **64** dient einer radialen Begrenzung einer (erneuten) Aufweitung des Federrings 58. Der Vorsprung 64 ist im unteren Bereich der Ringnut 62 angeordnet. Der Federring 58 fällt bei einer Montage der Knochenschraube 10 und zugleich lotrechter Ausrichtung der Knochenschraube der Schwerkraft folgend axial nach unten (= Fallring) und liegt dann an dem Vorsprung 64 an.

Der Federring 46 ist durch einen Seitenwandvorsprung **66** des Kopfgehäuses 18 gegenüber einem axialen Entfernen aus der Schraubenkopfaufnahme 64 des Kopfgehäuses 18 gesichert. Das Kopfgehäuse 18 ist damit über den Federring 58 unlösbar mit dem Schraubenkopf 16 verbunden. D.h. die Verbindung des Kopfgehäuses 18 mit dem Schraubenkopf 16 kann nicht zerstörungsfrei gelöst werden.

Die Knochenschraube kann einen oder mehrere in den Figuren 1, 2 und 4 wiedergegebene Sicherungsstifte 65 aufweisen, die einer axialen Lagesicherung des Federrings 58 am Kopfgehäuse 18 bzw. einer Begrenzung einer Axialbewegung des Federrings relativ zum Kopfgehäuse 18 dienen.

Das Einsatzteil 54 ist geneinsam mit dem Kopfgehäuse gegenüber der Gehäuselängsachse 20 verkippbar und über die Stabaufnahme 28 mit einem Drehwerkzeug (nicht gezeigt) zugänglich.

Der Gewindeschaft 12 der Knochenschraube 10 ist als Hohlschaft ausgebildet und kann im Bedarfsfall ein Bohrspitze aufweisen.

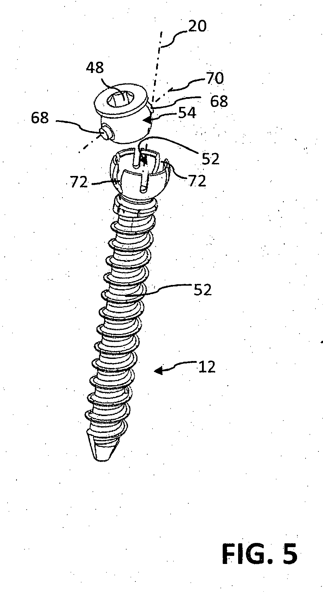

**Fig. 5** zeigt den Gewindeschaft 12 und das Einsatzteil 54 der vorstehend beschriebenen Knochenschraube in einer explodierten perspektivischen Ansicht.

Von dem Einsatzteil 54 stehen zwei erste Lagerzapfen **68** seitlich weg, die auf einer gemeinsamen Querachse **70** angeordnet sind. Die Lageraufnahme 52 des Schraubenkopfs 16 weist Seitenwandausnehmungen **72** zur Aufnahme der Lagerzapfen 68 auf. Die Seitenwandausnehmungen 72 sind einenends offen ausgebildet. Nach einem in der Zeichnung nicht näher wiedergegebenen Ausführungsbeispiel können die Seitenwandausnehmungen auch als nutartige Vertiefungen der Seitenwand der Lageraufnahme 52 ausgebildet sein.

Die beiden ersten Lagerzapfen 68 sind im Montagezustand der Knochenschraube in den Seitenwandausnehmungen 72 angeordnet, so dass das Einsatzteil 54 mit dem Schraubenkopf 16 bezüglich der Längsachse 22 der Knochenschraube 10 drehfest verkoppelt ist.

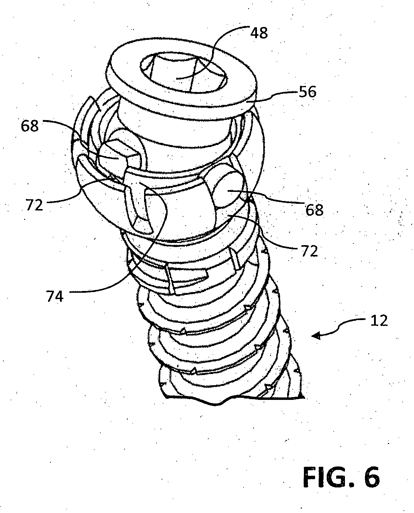

**In** **Fig. 6** ist eine alternative Lagerung des Einsatzteils 54 an dem Schraubenkopf 16 gezeigt. Das Einsatzteil 54 ist über einen (zusätzlichen) Kardanring **74** am Schraubenkopf 16 der Knochenschraube 12 verschwenkbar gelagert. Das Einsatzteil 54 ist mit seinen beiden ersten Lagerzapfen 68 in nach oben hin offenen Seitenwandausnehmungen 72 des Kardanrings 74 gehalten angeordnet. Der Kardanring 74 weist seinerseits ein Paar zweite Lagerzapfen 68 auf, die sich in die Seitenwandausnehmungen 72 des Schraubenkopfes 16 der Knochenschraube 10 hinein erstrecken.

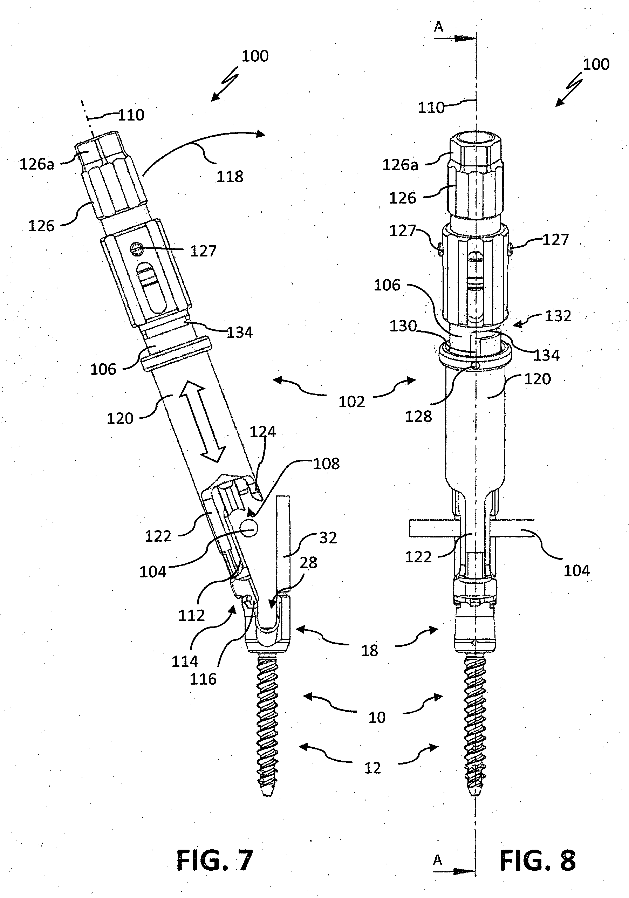

In den **Figuren 7** und **8** ist ein Knochenschraubensystem **100** mit einer vorstehend erläuterten Knochenschraube 10 und mit einem Repositionsinstrument **102** zum Einbringen und Fixieren eines Fixierstabs **104** in der Stabaufnahme 28 (Fig. 4) des Kopfgehäuses 18 der Knochenschraube 10 gezeigt.

Das Repositionsinstrument 102 weist eine Führungshülse **106** mit einer Durchgangsbohrung **108** auf, die sich entlang der Instrumentenlängsachse **110** des Repositionsinstruments 102 erstreckt. Die Führungshülse 106 ist mit einem Wandprofil **112** versehen, das sich entlang der Instrumentenlängsachse 110 des von der Führungshülse 106 wegerstreckt. Das Wandprofil 112 weist an seinem freien Ende **114** zweite Kopplungsmittel **116** auf, die zu den ersten Kopplungsmitteln 38 des ersten Wandabschnitts 30 des Kopfgehäuses 18 komplementär ausgebildet sind.

Das Repositionsinstrument 102 ist vorliegend mit den zweiten Kopplungsmitteln 116 des Wandprofils 112 an die ersten Kopplungsmittel 38 des Kopfgehäuses 18 der Knochenschraube 10 angekoppelt. Die zweiten Kopplungsmittel 116 ermöglichen eine in axialer Richtung zugfeste Verbindung zwischen dem Repositionsinstrument 102 und dem Kopfgehäuse 18.

Die zweiten Kopplungsmittel 116 sind vorliegend im Wesentlichen hakenförmig ausgeformt und hintergreifen in der gezeigten Ankoppelstellung des Repositionsinstruments 102 an dem Kopfgehäuse 18 der Knochenschraube 10 die ersten Kopplungsmittel 38 des ersten Wandschenkels 30 des Kopfgehäuses 18 in einer radialen und in einer axialen Richtung. Das Repositionsinstrument 102 ist somit in der gezeigten Ankoppelstellung in die beiden ersten Kopplungsmittel 38 des ersten Wandabschnitts 32 des Kopfgehäuses 18 eingehängt. Das Repositionsinstrument 102 kann gegenüber der Gehäuselängsachse 20 des Kopfgehäuses 18 in einer Schwenkrichtung **118** in seine nachstehend erläuterte Funktionsstellung am Kopfgehäuse 18 verschwenkt werden. Die ersten Kopplungsmittel 38 des Kopfgehäuses 18 dienen dem Repositionsinstrument 10 beim Ausführen der Schwenkbewegung dabei als Widerlager bzw. als Angelpunkt.

Auf der Führungshülse 106 ist eine Arretierhülse **120** angeordnet, die die Führungshülse 106 außenseitig (vollständig) umgreift. Die Arretierhülse 120 dient einem Arretieren des Repositionsinstruments 102 an dem Kopfgehäuse 18 in seiner vorgegebenen Funktionsstellung.

Die Arretierhülse 120 ist relativ zur Führungshülse 106 zwischen einer hier wiedergegebenen Freigabestellung und einer Arretierstellung auf der Führungshülse 106 axial verschiebbar angeordnet. Dies ist in Fig 7 durch den Doppelpfeil verdeutlicht.

Die Arretierhülse 120 weist einen Arretierarm **122** auf. Der Arretierarm erstreckt sich - in einer zu dem Wandprofil 112 der Führungshülse 106 entsprechenden Weise - von der Arretierhülse 120 in axialer Richtung weg. Der Arretierarm 122 greift in der Funktionsstellung des Repositionsinstruments 102 am Kopfgehäuse 18 und bei in ihrer Arretierstellung angeordneter Arretierhülse 120 in die in Fig. 3 gezeigte nutförmige Arretierausnehmung 50 des ersten Wandprofils 30 des Kopfgehäuses 18 ein.

Die Führungshülse 106 kann, wie dies in Fig. 7 gezeigt ist, an ihrem dem Arretierarm 122 gegenüberliegenden Umfangsabschnitt einen Arretierfortsatz **124** aufweisen. Der Arretierfortsatz erstreckt sich parallel zu dem Arretierarm von der Arretierhülse 120 in axialer Richtung weg.

An der Führungshülse 106 ist ein Drehknebel bzw. Drehwerkzeug **126** gelagert. Das Drehwerkzeug 126 weist einenends ein Antriebsprofil **126a** für den Angriff eines Schraubenschlüssels oder dergl. auf. Das Drehwerkzeug kann sich, wie dies bei dem in Fig. 7 gezeigten Ausführungsbeispiel der Fall ist, in die Durchgangsbohrung 108 der Führungshülse 106 zumindest abschnittsweise hineinerstrecken. Das Drehwerkzeug 126 ist entlang der Instrumentenlängsachse 110 gegenüber der Führungshülse 106 rotatorisch verstellbar. Bei dem gezeigten Ausführungsbeispiel ist das Drehwerkzeug 126 an der Führungshülse 106 axial lagefixiert. Zum axial unverschieblichen Festsetzen des Drehwerkzeugs 126 an der Führungshülse 106 dienen zwei Sicherungsstifte **127**, die die Führungshülse in radialer Richtung durchgreifen und deren Enden in eine Ringnut (nicht gezeigt) des Drehwerkzeugs 126 eingreifen. Das Drehwerkzeug 126 ist hohlzylindrisch ausgeführt.

Wie aus Fig. 8 hervorgeht, weist die Arretierhülse 120 einen Führungsstift **128** auf. Der Führungsstift 128 ragt innenseitig in radialer Richtung von der Arretierhülse 120 weg und greift in einen Längsabschnitt **130** einer Führungsnut **132** der Führungshülse 106 ein. Die Arretierhülse 120 ist über den Führungsstift an der Führungshülse geführt und im Bereich des Längsabschnitts 130 der Führungsnut 132 mit dieser drehfest verbunden. Der Längsabschnitt 130 der Führungsnut 132 ist über einen in Umfangsrichtung verlaufenden Nutabschnitt **134** mit einem zweiten Längsabschnitt (nicht gezeigt) der Führungsnut 132 verbunden. Der zweite Längsabschnitt der Führungsnut 132 ist zu dem in der Figur unteren Ende (nicht gezeigt) der Führungshülse 106 hin offen ausgebildet, um ein Einführen des Führungsstifts 128 in die Führungsnut 132 beim Auffädeln (Aufschieben) der Arretierhülse 120 auf die Führungshülse 106 während des Zusammenbaus des Repositionsinstruments 102 bzw. ein Abziehen der Arretierhülse 120 von der Führungshülse 106 bei der Demontage des Repositionsinstruments zu ermöglichen.

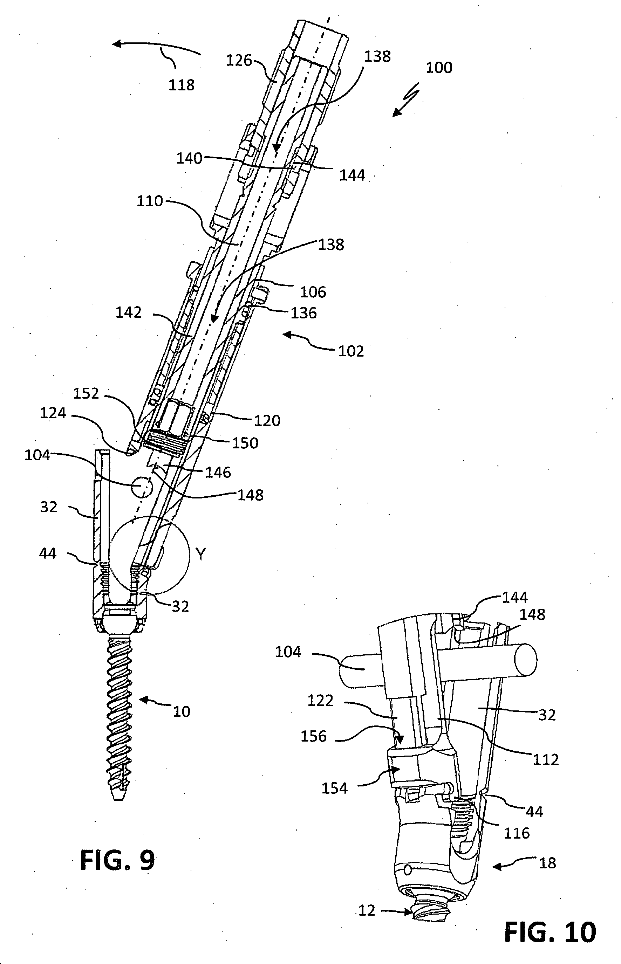

In **Fig. 9** ist ein Knochenschraubensystem 100 im Längsschnitt wiedergegeben, das im Wesentlichen dem in den Figuren 7 und 8 gezeigten Knochenschraubensystem entspricht. Die Arretierhülse 120 ist über ein an der Führungshülse 106 abgestütztes Federelement **136** in ihrer Arretierstellung vorgespannt gehalten.

Das Drehwerkzeug 126 weist einen Werkzeugdurchgang (=Durchgangsbohrung) **138** mit einem Innengewinde **140** auf, in das ein Druckglied **142** mit einem Außengewinde **144** eingreift. Das Druckglied 142 ist durch diese Gewindepaarung mittels einer um die Instrumentenlängsachse **110** gerichteten Schraubbewegung des Drehwerkzeugs 126 gegenüber der Führungshülse axial verstellbar. Das Drehwerkzeug 126 ist somit funktionell als eine Bewegungsmutter ausgebildet. Das Druckglied 142 weist ebenfalls einen Werkzeugdurchgang (Durchgangsbohrung) 138 auf.

Das Druckglied weist einenends zwei zungenförmige Fortsätze **146** auf, von denen in Fig. 9 aus Darstellungsgründen nur ein zungenförmiger Fortsatz 146 wiedergegeben ist. Die beiden Fortsätze 146 sind voneinander beabstandet angeordnet und zueinander im Wesentlichen parallel oder parallel verlaufend ausgerichtet. Die beiden Fortsätze weisen jeweils eine konkave Anlagefläche **148** für den Fixierstab 104 auf. Die Anlageflächen 148 können auch in anderer Weise, beispielsweise plan, ausgeführt sein.

Das Druckglied weist einen Federring **150** auf, der als Haltemittel für die Fixierschraube **152** dient. Die Fixierschaube 152 ist im Federring 150 im Klemmsitz gehalten Angeordnet und durch diesen am Repositionswerkzeug 102 lösbar gehalten. Der Federring 150 ist in einer Ringnut (nicht gezeigt) des Druckglieds 142 am Druckglied 142 axial lagegesichert.

**Fig. 10** zeigt die Knochenschraube 10 mit dem daran angekoppelten Repositionsinstrument 102 in einer ausschnittsweise vergrößerten Darstellung.

Die Führungshülse 106 weist einen seitlichen Wandvorsprung **154** auf, der mit einer Führungsausnehmung **156** für den Arretierarm 122 versehen ist. Der Arretierarm 122 erstreckt sich durch die Führungsausnehmung 156 hindurch und ist darin verschiebbar geführt.

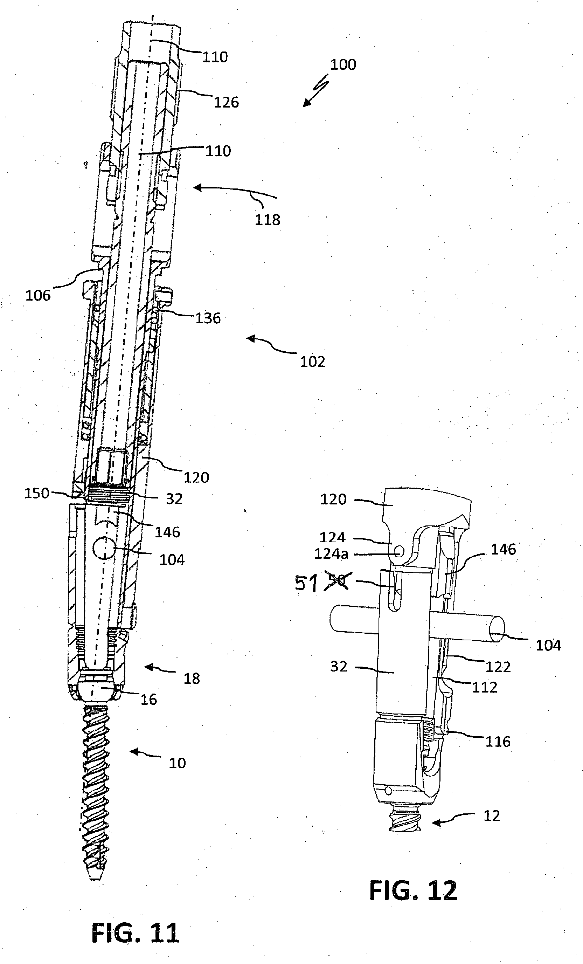

In **Fig. 11** ist das Repositionsinstrument 102 aus der in Fig. 12 gezeigten Ankoppelstellung gegenüber dem Kopfgehäuse 18 in Richtung auf die Kopfgehäuselängsachse bzw. seine Funktionsstellung verschwenkt angeordnet. Das Repositionswerkzeug 102 und das Kopfgehäuse 18 der Knochenschraube sind in Richtung der Instrumentenlängsachse zueinander nahezu fluchtend angeordnet.

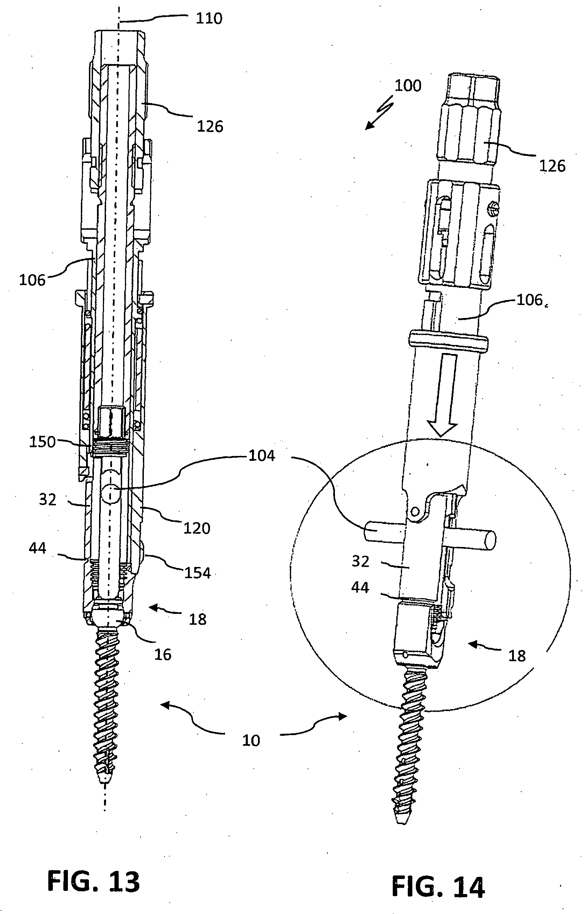

Die Arretierhülse 120 wurde manuell axial in Richtung auf das Drehwerkzeug 126 gegenüber der Führungshülse verschoben (zurückgezogen) und ist in ihrer Freigabeposition angeordnet. In der Freigabeposition ist ein Verschwenken des Repositionsinstruments 102 relativ zum Kopfgehäuse 18 der Knochenschraube 10 in die bzw. aus der in Fig. 13 gezeigten Funktionsstellung des Repositionsinstruments 102 ermöglicht.

Der Fixierstab 104 wurde durch das Verschwenken des Repositionsinstruments mittels des Wandprofils 112 der Führungshülse in Richtung auf den zweiten Wandschenkel 32 des Kopfgehäuses 18 bewegt (gehebelt). Der Fixierstab liegt an dem Wandprofil 112 des Repositionsinstruments 102 an und ist abschnittsweise zwischen dem zweiten Wandschenkel 32 und dem Wandprofil 112 des Repositionsinstruments 102 angeordnet.

In **Fig. 12** ist das Kopfgehäuse 18 und das untere Ende des Repositionsinstruments 102 in einer ausschnittsweise vergrößerten Darstellung wiedergegeben. Der zweite Wandschenkel 32 des Kopfgehäuses 18 weist vorliegend im Gegensatz zu dem in den Figuren 7 und 8 gezeigten Knochenschraube zusätzlich eine zweite Arretierausnehmung **51** für den Arretierfortsatz 124 der Arretierhülse 120 auf. Der Arretierfortsatz 124 des Repositionsinstruments 102 weist im Gegensatz zu dem Instrument gemäß Fign. 7 und 8 einen Arretierstift 124a auf, der sich von dem Arretierfortsatz 124 in radialer Richtung nach innen wegerstreckt. Der Arretierstift 124a ist dazu ausgebildet, in der Arretierstellung der am Kopfgehäuse 18 arretierten Arretierhülse 120 in die Arretierausnehmung 51 des zweiten Wandschenkels 32 einzugreifen.

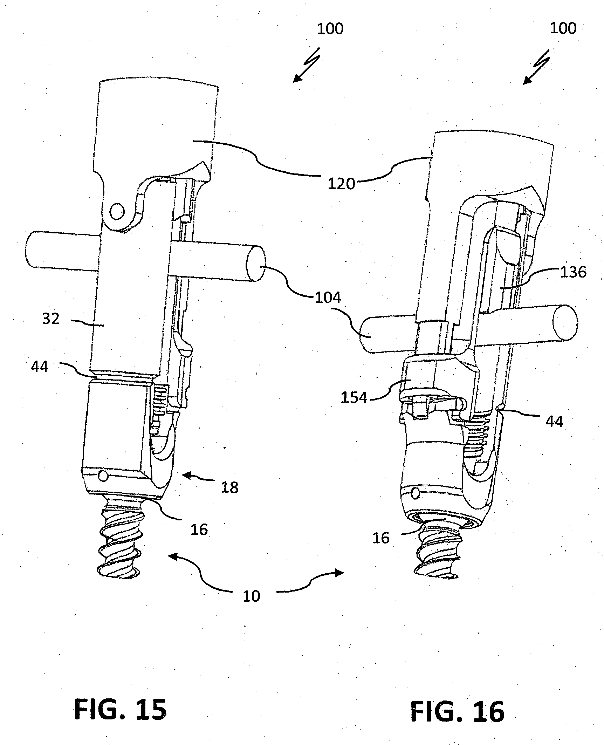

In den **Figuren 13** bis **16** ist das Repositionswerkzeug 102 gemäß Fig. 9 am Kopfgehäuse der Knochenschraube 10 in seiner Funktionsstellung arretiert. Die Arretierhülse 120 ist in Arretierstellung angeordnet und übergreift den ersten und den zweiten Wandschenkel 30, 32 des Kopfgehäuses 18 umfangsseitig (von außen). Die Führungshülse 106 des Repositionsinstruments 102 ist an dem Kopfgehäuse 18 der Knochenschraube 10 drehfest und axial unverschieblich angekoppelt. Der Arretierstift 124a greift in die Arretierausnehmung 51 des zweiten Wandschenkels 32 ein.

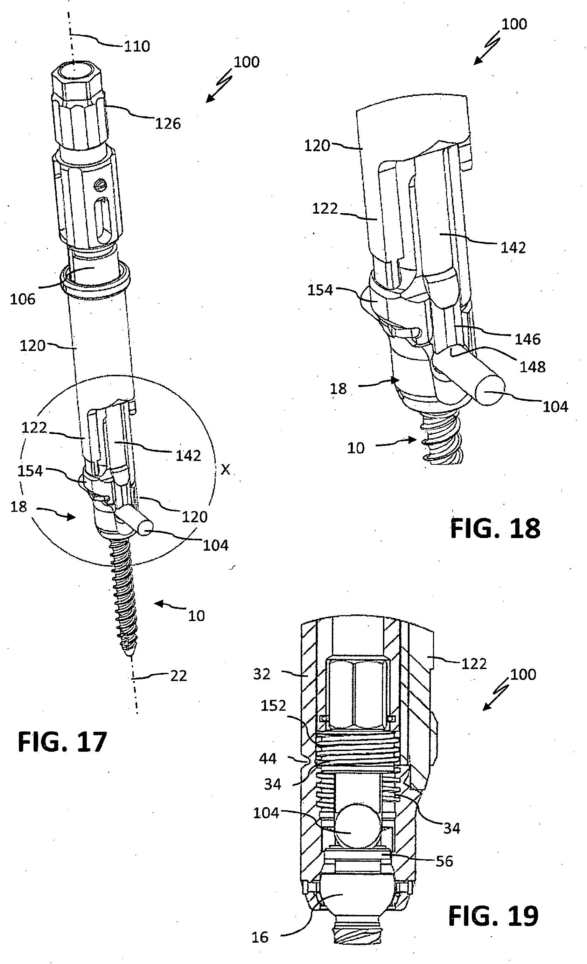

In den Figuren **17** bis **19** ist das Knochenschraubensystem 102 in einem weiteren Betriebszustand gezeigt. Das Druckglied 142 wurde durch Verdrehen des Drehwerkzeugs 126 um die Instrumentenlängsachse 110 axial in Richtung auf das Kopfgehäuse 18 verstellt. Der Fixierstab 104 liegt, wie dies insbesondere in Fig. 18 gezeigt ist, an den beiden gewölbten Anlageflächen 148 der zungenartigen Fortsätze 146 des Druckglieds 142 an und ist in der Stabaufnahme 28 des Kopfgehäuses 18 positioniert.

Aus der ausschnittsweisen Schnittdarstellung in Fig. 19 geht hervor, dass die Fixierschraube 152 in diesem Betriebszustand des Repositionswerkzeugs 102 ohne Kontakt zu dem Fixierstab 104 ist. Die Fixierschraube 152 ist zu den beiden Innengewindesegmenten 34 der Wandschenkel 30, 32 des Kopfgehäuses in axialer Richtung unmittelbar benachbart angeordnet und kann darin ohne Kraftsaufwand eingeschraubt werden.

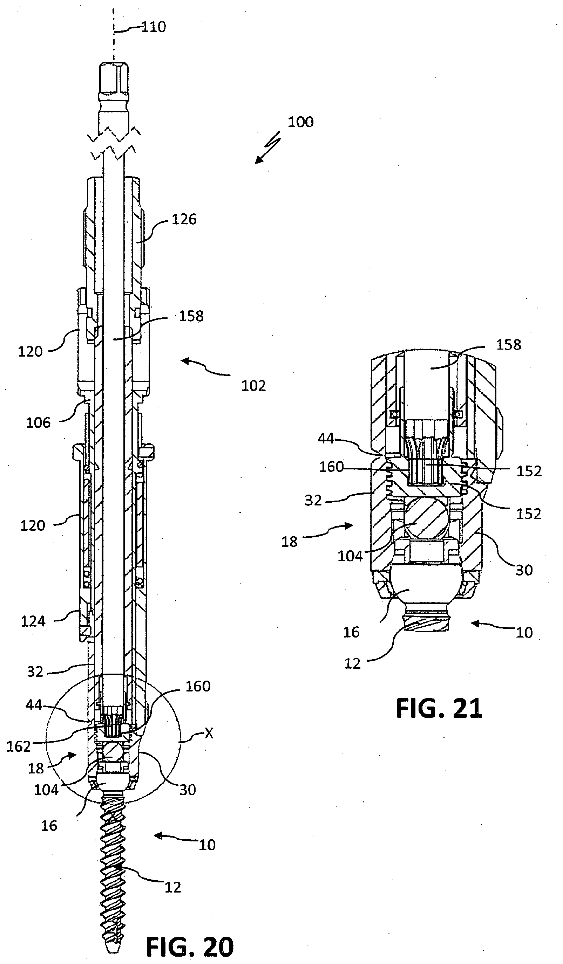

Hierzu wird ein stangenförmiger Schraubendreher **158** eingesetzt, der in den **Figuren 20** und **21** wiedergegeben ist. Der Schraubendreher 158 ist in die Durchgangsbohrungen des Drehwerkzeug und des Druckglieds eingeführt. Der Schraubendreher 158 weist einenends eine auf das Antriebsprofil **160** der Fixierschraube 152 ausgerichtetes Schrauben-Mitnahmeprofil **162**, hier ein sogenanntes Kronenprofil (beispielsweise ein Torxprofil) auf. Das Schrauben-Mitnahmeprofil 162 ist in das Antriebsprofil 160 der Fixierschraube eingeführt. Die Fixierschraube 152 wurde mit Hilfe des Schraubendrehers 158 in die Innengewindesegmente 34 der beiden Wandschenkel 30, 32 bis zu ihrer gezeigten Fixierstellung eingedreht. In der Fixierstellung ist der Fixierstab 104 in der Stabaufnahme 28 des Kopfgehäuses 18 zwischen der Fixierschraube 152 und dem Schraubenkopf 16 geklemmt gehalten angeordnet. Der Schraubendreher 158 weist anderenends ein Vierkantprofil für den Ansatz eines Schraubenschlüssels oder dergl. auf. Es versteht sich, das der Schraubendreher 158 auch ein anderes Antriebs- und/oder Mitnahmeprofil 160, 162 aufweisen kann.

Das Kopfgehäuse 18 ist am Schraubenkopf 16 der Knochenschraube 10 festgesetzt. D.h. das Kopfgehäuse 18 ist am Schraubenkopf 16 im Klemmsitz gehalten angeordnet.

Nachfolgend wird die Funktion des Knochenschraubensytems unter Bezugnahme auf die Figuren erläutert:
Nach einem Einschrauben der Knochenschraube 10 in den Wirbelkörper oder den Pedikel eines Wirbels (nicht gezeigt) wird das Kopfgehäuse 18 derart gegenüber der Längsachse 22 des Gewindeschafts der Knochenschraube 18 verdreht, dass der Fixierstab 104 innenseitig des zweiten Wandschenkels 32 des Kopfgehäuses 18 zu liegen kommt.

Nachfolgend wird das Repositionsinstrument 102 an den ersten Wandschenkel 30 des Kopfgehäuses 18 angekoppelt bzw. angesetzt, so dass die ersten Kopplungsmittel 38 des Wandschenkels 30 und die zweiten Kopplungsmittel 116 des Repositionsinstruments 102 ineinandergreifen bzw. miteinander verhakt sind. Das Repositionsinstrument 102 wird nachfolgend gegenüber dem Kopfgehäuse 18 bis zum Erreichen der Funktionsstellung (Fign. 13 bis 21) des Repositionswerkzeugs 102 verschwenkt. Der Fixierstab 104 wird dabei mit Hilfe des Wandprofils 112 des Repositionswerkzeugs 102 ohne zusätzliches Werkzeug in radialer Richtung auf den zweiten Wandschenkel 32 des Kopfgehäuses 18 der Knochenschraube 10 zubewegt bzw. gehebelt. Die Arretierhülse 120 wird vor dem Erreichen der Funktionsstellung des Repositionswerkzeugs relativ zum Kopfgehäuse 18 manuell und entgegen der Kraft des Federelements 136 in axialer Richtung in seine Freigabestellung (Fign. 11, 12) gezogen. Nach dem Erreichen der Funktionsstellung des Repositionswerkzeugs 102 wird die Arretierhülse 120 freigegeben, so dass diese durch die Kraft des Federelements 136 selbsttätig in axialer Richtung relativ zur Führungshülse 106 in seine Arretierstellung überführt wird. Das Repositionswerkzeug 102 ist durch die Arretierhülse in seiner Funktionsstellung am Kopfgehäuse 18 der Knochenschraube 10 arretiert. Der Fixierstab 104 ist zwischen dem Wandprofil 112 des Repositionsinstruments 102 und dem (langen) zweiten Wandschenkel 32 des Kopfgehäuses 18 gefangen.

Nachfolgend wird das Drehwerkzeug 126 manuell oder mittels eines am Antriebsprofil des Drehwerkzeugs angreifenden weiteren Werkzeugs gegenüber der Führungshülse 116 (händisch) verdreht. Das Druckglied 142 wird dadurch gegenüber der Führungshülse 116 in axialer Richtung verstellt. Der Fixierstab 104 wird durch das Druckglied 142 in axialer Richtung auf und gegen den Schraubenkopf 16 bzw. dessen Einsatzteil 54 (Fig. 4) gedrückt. Nachfolgend wird der Schraubendreher 158 in axialer Richtung in das Repositionswerkzeug 102 eingeführt. Die Fixierschraube und die Innengewindesegmente 34 der Wandschenkel 30, 32 werden unter einem sanftem axialgerichteten Druck und durch Drehen des Schraubendrehers in einen gegenseitigen Gewindeeingriff gebracht. Sobald die Fixierschraube 152 in die Innengewindesegmente 34 der Stabaufnahme 28 eingreift, wird die Fixierschraube 152, vorzugsweise mit einem definierten Drehmoment, in die Innengewindesegmente 34 eingeschraubt. Der Fixierstab 104 wird durch ein entsprechendes Anzugsmoment der Fixierschraube 152 in der Stabaufnahme 28 festgesetzt und so das Kopfgehäuse 18 zugleich in seiner optimalen Stellung relativ zur Längsachse 22 der Knochenschraube 18 am Schraubenkopf 16 festgeklemmt (festgesetzt). Nachfolgend wird der Schraubendreher 158 aus dem Repositionswerkzeug 102 herausgezogen. Das Druckglied 142 wird durch ein Drehen des Drehwerkzeugs um die Instrumentenlängsachse 110 in axialer Richtung von dem Fixierstab zurückbewegt. Nachfolgend kann die Arretierhülse 120 in ihre Freigabestellung überführt werden. Das Repositionswerkzeug 102 kann nachfolgend aus seiner Funktionsstellung am Kopfgehäuse gegenüber dem Kopfgehäuse 18 in die Ankoppelstellung (Fig. 7) zurück verschwenkt und vom Kopfgehäuse 18 abgekoppelt werden. Der zweite Wandschenkel des Kopfgehäuses wird nachfolgend auf die Länge L1 des ersten Wandschenkels gekürzt. Dies kann beispielsweise durch Abbrechen des freien Endabschnitts des Wandschenkels im Bereich der Sollbruchstelle 44 des zweiten Wandschenkel erfolgen.

## Patentansprüche

1. Knochenschraubensystem (100) mit
- einer Knochenschraube (10) für Implantate zur Korrektur und Stabilisierung der Wirbelsäule, umfassend:
- einen Gewindeschaft (12) mit einem Schraubenkopf (16), der ein Antriebsprofil (48) für ein Drehwerkzeug aufweist, und
- ein Kopfgehäuse (18), das über einen Kopplungsabschnitt (24) mit dem Schraubenkopf (16) verbunden ist, wobei das Kopfgehäuse (18) eine Stabaufnahme (28) für einen Fixierstab (104) aufweist, die von einem ersten und einem zweiten Wandschenkel (30, 32) seitlich begrenzt ist,
wobei sich die beiden Wandschenkel in Richtung einer Gehäuselängsachse (20) vom Kopplungsabschnitt (24) wegerstrecken;
wobei die beiden Wandschenkel (30, 32) jeweils ein Innengewindesegment (34) für eine Fixierschraube (152) aufweisen, mittels derer der Fixierstab in der Stabaufnahme festlegbar ist,
wobei der zweite Wandschenkel (32) den ersten Wandschenkel (30) in axialer Richtung überragt,
wobei der erste Wandschenkel (30) an seinem freien Randabschnitt (36) ein erstes Kopplungsmittel (38) zum Ankoppeln eines Repositionsinstruments (102) aufweist und
wobei der zweite Wandschenkel (32) eine Sollbruchstelle (44) aufweist, die axial auf Höhe oder im Wesentlichen auf Höhe des freien Endes (42) des ersten Wandschenkels (30) angeordnet ist; und
- einem Repositionsinstrument (102) zum Einbringen und Fixieren eines Fixierstabs (104) in der Stabaufnahme (28) des Kopfgehäuses (18) der Knochenschraube (10), wobei das Repositionsinstrument folgendes umfasst:
- eine Führungshülse (106) mit einer längsaxialen Durchgangsbohrung (108) und mit einem Wandprofil (112), das sich in axialer Richtung von der Führungshülse (106) wegerstreckt, wobei das Wandprofil (112) an seinem freien Ende ein zweites Kopplungsmittel (116) aufweist, das zu dem ersten Kopplungsmittel (38) des ersten Wandschenkels (30) des Kopfgehäuses (18) der Knochenschraube (10) komplementär ausgebildet ist;
- ein Arretiermittel (120) zum Arretieren der Führungshülse (106) an den beiden Wandschenkeln (30, 32) des Kopfgehäuses (18);
- ein Druckglied (142) zum Absenken des Fixierstabs (104), das in der Durchgangsbohrung (108) der Führungshülse (106) axial verstellbar angeordnet ist und;
- ein Drehwerkzeug (126), das an der Führungshülse (106) relativ zur Führungshülse (106) verdrehbar gelagert ist, wobei das Drehwerkzeug (126) mit dem Druckglied (142) derart gekoppelt ist, dass das Druckglied (142) durch ein Verdrehen des Drehwerkzeugs (126) relativ zu der Führungshülse (106) axial verstellbar ist, wobei das Arretiermittel eine Arretierhülse (120) ist, die die Führungshülse (106) umgreift und die in axialer Richtung zwischen einer Freigabestellung und einer Arretierstellung relativ zur Führungshülse (106) verstellbar ist, wobei die Arretierhülse in ihrer Arretierstellung den ersten Wandschenkel (30) und den zweiten Wandschenkel (32) des Kopfgehäuses (18) der Knochenschraube (10) außenseitig übergreift und wobei die Arretierhülse einen Arretierarm (122) aufweist, der sich von der Arretierhülse (120) in axialer Richtung wegerstreckt und der dazu ausgebildet ist, in der Arretierstellung der Arretierhülse (120) in eine Arretierausnehmung (50) des ersten Wandschenkels (30) der Knochenschraube (10) einzugreifen.

2. Knochenschraubensystem nach Anspruch 1, **dadurch gekennzeichnet, dass** die Arretierhülse (120) einen Arretierfortsatz (124) aufweist, der sich an einem von dem Arretierarm (122) gegenüberliegen Hülsenabschnitt der Arretierhülse (120) von der Arretierhülse (120) in axialer Richtung wegerstreckt und der dazu ausgebildet ist, das zweite Wandprofil (32) der Knochenschraube (10) in der Arretierstellung der Arretierhülse (120) außenseitig zu übergreifen.

3. Knochenschraubensystem nach Anspruch 1 oder 2, **dadurch gekennzeichnet, dass** die Führungshülse (106) eine Führungsausnehmung (156) aufweist, in der der Arretierarm (122) der Arretierhülse (120) verschiebbar geführt ist.

4. Knochenschraubensystem nach einem der vorhergehenden Ansprüche, **dadurch gekennzeichnet, dass** das Arretiermittel (120) an der Führungshülse (106) in Arretierstellung vorgespannt gehalten ist.

5. Knochenschraubensystem nach einem der vorhergehenden Ansprüche, **dadurch gekennzeichnet, dass** das Drehwerkzeug (126) an der Führungshülse (106) axial lagefixiert ist und mit dem Druckglied (142) in einem lösbaren Gewindeeingriff steht.

6. Knochenschraubensystem nach einem der vorhergehenden Ansprüche, **dadurch gekennzeichnet, dass** das Drehwerkzeug (126) und das Druckglied (142) des Repositionsinstruments (102) jeweils einen Werkzeugdurchgang für einen Schraubendreher (158) zum Einschrauben der Fixierschraube (152) in das Kopfgehäuse (18) der Pedikelschraube (10) aufweist.

7. Knochenschraubensystem nach einem der vorhergehenden Ansprüche, **dadurch gekennzeichnet, dass** das Druckglied (142) des Repositionsinstruments (102) ein Federelement, bevorzugt einen Federring (150), zum Halten der Fixierschraube (152) aufweist.

8. Knochenschraubensystem nach einem der vorhergehenden Ansprüche, **dadurch gekennzeichnet, dass** die Sollbruchstelle (44) des zweiten Wandschenkels (32) der Knochenschraube (10) zumindest eine Kerbe des zweiten Wandschenkels (32) umfasst.

9. Knochenschraubensystem nach einem der vorhergehenden Ansprüche, **dadurch gekennzeichnet, dass** sich das Innengewindesegment (34) des ersten Wandschenkels (30) der Knochenschraube (10) in axialer Richtung bis zum freien Ende (42) des ersten Wandschenkels (30) und dass sich das Innengewindesegment (34) des zweiten Wandschenkel (32) bis zu der Sollbruchstelle (44) erstreckt.

10. Knochenschraubensystem nach einem der vorhergehenden Ansprüche, **dadurch gekennzeichnet, dass** das Kopfgehäuse (18) am Schraubenkopf (16) relativ zur Längsachse (22) des Gewindeschafts (12) der Knochenschraube (10) auslenkbar angeordnet und/oder um seine Gehäuselängsachse (20) frei drehbar am Schraubenkopf (16) angeordnet ist.

11. Knochenschraubensystem nach einem der vorhergehenden Ansprüche, **dadurch gekennzeichnet, dass** das Antriebsprofil (48) von einem Einsatzteil (54) gebildet ist, das in einer Lageraufnahme (52) des Schraubenkopfs (16) angeordnet ist, wobei das Einsatzteil (54) mit dem Schraubenkopf (16) in Richtung um die Längsachse (22) des Gewindeschafts (12) der Knochenschraube (10) drehfest verkoppelt ist und wobei das Einsatzteil (54) am Kopfgehäuse (18) geführt gehalten und gemeinsam mit diesem gegenüber der Längsachse des Gewindeschafts (22) auslenkbar ist.

12. Knochenschraubensystem nach einem der vorhergehenden Ansprüche, **dadurch gekennzeichnet, dass** das Kopfgehäuse (18) über einen, vorzugsweise geschlitzten, Federring (58) mit dem Schraubenkopf (16) verbunden ist, wobei das Kopfgehäuse (18) innenseitig eine Ringnut (62) und innerhalb der Ringnut (62) einen Vorsprung (64) für eine radiale Begrenzung einer Aufweitung des Federrings (58) aufweist.

## Claims

1. Bone screw system (100) with
- a bone screw (10) for implants for correcting and stabilizing the spinal column, comprising:
- a threaded shank (12) with a screw head (16), which has a driving profile (48) for a turning tool, and
- a head housing (18), which is connected to the screw head (16) via a coupling portion (24), wherein the head housing (18) has a rod recess (28) for a fixing rod (104), which rod recess (28) is laterally delimited by a first and a second wall leg (30, 32), wherein the two wall legs extend away from the coupling portion (24) in the direction of a housing longitudinal axis (20);
wherein the two wall legs (30, 32) each have an internal thread segment (34) for a fixing screw (152), by means of which the fixing rod can be secured in the rod recess,
wherein the second wall leg (32) protrudes axially beyond the first wall leg (30),
wherein the first wall leg (30) has, at the free edge portion (36) thereof, a first coupling means (38) for coupling a repositioning instrument (102), and
wherein the second wall leg (32) has a predetermined breaking point (44), which is arranged axially at the height of or substantially at the height of the free end (42) of the first wall leg (30); and
- a repositioning instrument (102) for introducing and fixing a fixing rod (104) in the rod recess (28) of the head housing (18) of the bone screw (10), wherein the repositioning instrument comprises the following:
- a guide sleeve (106) with a longitudinal axial through-bore (108) and with a wall profile (112) which extends away from the guide sleeve (106) in the axial direction, wherein the wall profile (112), has, at its free end, a second coupling means (116) which is designed complementing the first coupling means (38) of the first wall leg (30) of the head housing (18) of the bone screw (10);
- a locking means (120) for locking the guide sleeve (106) on the two wall legs (30, 32) of the head housing (18) ;
- a pressure member (142) for lowering the fixing rod (104) which is arranged in an axially adjustable manner in the through-bore (108) of the guide sleeve (106); and
- a turning tool (126) which is mounted on the guide sleeve (106) in a manner rotatable relative to the guide sleeve (106), wherein the turning tool (126) is coupled to the pressure member (142) in such a way that the pressure member (142) is axially adjustable by turning the turning tool (126) relative to the guide sleeve (106),
wherein the locking means is a locking sleeve (120), which engages around the guide sleeve (106) and which is adjustable in the axial direction relative to the guide sleeve (106) between a release positon and a locking position, wherein the locking sleeve in its locking positon engages over the outside of the first wall leg (30) and the second wall leg (32) of the head housing (18) of the bone screw (10), and wherein the locking sleeve has a locking arm (122) which extends axially away from the locking sleeve (120) and which is designed, in the locking position of the locking sleeve (120), to engage in a locking recess (50) of the first wall leg (30) of the bone screw (10).

2. Bone screw system according to Claim 1, **characterized in that** the locking sleeve (120) has a locking extension (124) which extends axially away from a sleeve portion of the locking sleeve (120) lying opposite the locking arm (122) and is designed to engage over the outside of the second wall profile (32) of the bone screw (10) in the locking position of the locking sleeve (120).

3. Bone screw system according to Claim 1 or 2, **characterized in that** the guide sleeve (106) has a guide recess (156) in which the locking arm (122) of the locking sleeve (120) is guided displaceably.

4. Bone screw system according to one of the preceding claims, **characterized in that** the locking means (120) is held pretensioned on the guide sleeve (106) in the locking position.

5. Bone screw system according to one of the preceding claims, **characterized in that** the turning tool (126) is axially fixed in position on the guide sleeve (106) and is in a releasable threaded engagement with the pressure member (142).

6. Bone screw system according to one of the preceding claims, **characterized in that** the turning tool (126) and the pressure member (142) of the repositioning instrument (102) each have a tool passage for a screwdriver (158) for screwing the fixing screw (152) into the head housing (18) of the pedicle screw (10) .

7. Bone screw system according to one of the preceding claims, **characterized in that** the pressure member (142) of the repositioning instrument (102) has a spring element, preferably a spring ring (150), for retaining the fixing screw (152).

8. Bone screw system according to one of the preceding claims, **characterized in that** the predetermined breaking point (44) of the second wall leg (32) of the bone screw (10) comprises at least one notch of the second wall leg (32).

9. Bone screw system according to one of the preceding claims, **characterized in that** the internal thread segment (34) of the first wall leg (30) of the bone screw (10) extends axially as far as the free end (42) of the first wall leg (30), and **in that** the internal thread segment (34) of the second wall leg (32) extends as far as the predetermined breaking point (44).

10. Bone screw system according to one of the preceding claims, **characterized in that** the head housing (18) on the screw head (16) is displaceable relative to the longitudinal axis (22) of the threaded shank (12) of the bone screw (10) and/or is arranged on the screw head (16) so as to be freely rotatable about its housing longitudinal axis (20).

11. Bone screw system according to one of the preceding claims, **characterized in that** the driving profile (48) is formed by an insert part (54) which is arranged in a bearing recess (52) of the screw head (16), wherein the insert part (54) is coupled to the screw head (16) for conjoint rotation in a direction about the longitudinal axis (22) of the threaded shank (12) of the bone screw (10), and wherein the insert part (54) is held and guided on the head housing (18) and, together with the latter, is displaceable with respect to the longitudinal axis of the threaded shank (22).

12. Bone screw system according to one of the preceding claims, **characterized in that** the head housing (18) is connected to the screw head (16) via a preferably slotted spring ring (58), wherein the head housing (18) has, on the inside, an annular groove (62) and, inside the annular groove (62), a projection (64) for radially limiting an expansion of the spring ring (58).

## Revendications

1. Système (100) à vis ostéochirurgicale, comprenant
- une vis ostéochirurgicale (10) dévolue à des implants de correction et de stabilisation de la colonne vertébrale, comportant :
- une tige filetée (12), associée à une tête (16) de ladite vis qui est munie d'un profil d'entraînement (48) destiné à un outil rotatif, et
- un réceptacle frontal (18) relié à ladite tête (16) de la vis par l'intermédiaire d'un tronçon d'accouplement (24), ledit réceptacle frontal (18) étant doté d'un logement (28) qui est assigné à une broche (104) de blocage à demeure et est délimité, dans le sens latéral, par des première et seconde ailes de cloisonnement (30, 32), les deux ailes de cloisonnement s'étendant à partir dudit tronçon d'accouplement (24) dans la direction d'un axe longitudinal (20) dudit réceptacle ;
les deux ailes de cloisonnement (30, 32) étant respectivement pourvues d'un segment (34) à filetage intérieur, dédié à une vis (152) de blocage à demeure au moyen de laquelle ladite broche de blocage à demeure peut être assujettie dans le logement assigné à ladite broche,
la seconde aile de cloisonnement (32) dépassant au-delà de la première aile de cloisonnement (30) dans la direction axiale,
ladite première aile de cloisonnement (30) présentant, dans sa région marginale libre (36), un premier moyen d'accouplement (38) destiné à l'accouplement d'un instrument de repositionnement (102) et
ladite seconde aile de cloisonnement (32) comportant une zone (44) de rupture par destination située, dans le sens axial, à la hauteur ou pour l'essentiel à la hauteur de l'extrémité libre (42) de ladite première aile de cloisonnement (30) ; et
un instrument de repositionnement (102), dévolu à l'introduction et au verrouillage d'une broche (104) de blocage à demeure dans le logement (28) assigné à ladite broche dans le réceptacle frontal (18) de ladite vis ostéochirurgicale (10), sachant que ledit instrument de repositionnement inclut :
- une douille de guidage (106), pourvue d'un alésage longitudinal axial de passage (108) et d'un profil de paroi (112) partant de ladite douille de guidage (106) dans la direction axiale, ledit profil de paroi (112) présentant, à son extrémité libre, un second moyen d'accouplement (116) qui offre une réalisation complémentaire de celle du premier moyen d'accouplement (38) de la première aile de cloisonnement (30) du réceptacle frontal (18) de ladite vis ostéochirurgicale (10) ;
- un moyen d'arrêt (120), destiné à arrêter la douille de guidage (106) au niveau des deux ailes de cloisonnement (30, 32) dudit réceptacle frontal (18) ;
- un organe de pression (142) conçu pour faire descendre la broche (104) de blocage à demeure et logé, à déplacement axial, dans l'alésage de passage (108) de ladite douille de guidage (106) ; et
- un outil rotatif (126) monté sur la douille de guidage (106) avec faculté de rotation par rapport à ladite douille de guidage (106), ledit outil rotatif (126) étant accouplé avec l'organe de pression (142) de façon telle que ledit organe de pression (142) puisse être déplacé, dans le sens axial, sous l'effet d'une rotation dudit outil rotatif (126) par rapport à ladite douille de guidage (106),
le moyen d'arrêt étant une douille d'arrêt (120) ceinturant la douille de guidage (106) et pouvant être déplacée par rapport à ladite douille de guidage (106), dans la direction axiale, entre une position de libération et une position d'arrêt, sachant que, dans sa position d'arrêt, ladite douille d'arrêt coiffe extérieurement la première aile de cloisonnement (30) et la seconde aile de cloisonnement (32) du réceptacle frontal (18) de la vis ostéochirurgicale (10), et sachant que la douille d'arrêt comporte un bras d'arrêt (122) qui s'étend à partir de ladite douille d'arrêt (120) dans la direction axiale et est réalisé de manière à pénétrer, dans la position d'arrêt de ladite douille d'arrêt (120), dans un évidement d'arrêt (50) de ladite première aile de cloisonnement (30) de ladite vis ostéochirurgicale (10).

2. Système à vis ostéochirurgicale, selon la revendication 1, **caractérisé par le fait que** la douille d'arrêt (120) présente un appendice d'arrêt (124) qui s'étend à partir de ladite douille d'arrêt (120) dans la direction axiale, au niveau d'un tronçon de ladite douille d'arrêt (120) pointant à l'opposé du bras d'arrêt (122), et est réalisé de manière à coiffer extérieurement la seconde aile de cloisonnement (32) de ladite vis ostéochirurgicale (10) dans la position d'arrêt de ladite douille d'arrêt (120).

3. Système à vis ostéochirurgicale, selon la revendication 1 ou 2, **caractérisé par le fait que** la douille de guidage (106) présente un évidement de guidage (156) dans lequel le bras d'arrêt (122) de la douille d'arrêt (120) est guidé à coulissement.

4. Système à vis ostéochirurgicale, selon l'une des revendications précédentes, **caractérisé par le fait que** le moyen d'arrêt (120) est maintenu précontraint sur la douille de guidage (106) en position d'arrêt.

5. Système à vis ostéochirurgicale, selon l'une des revendications précédentes, **caractérisé par le fait que** l'outil rotatif (126) est bloqué à demeure sur la douille de guidage (106), dans le sens axial, et est amoviblement en prise par filetage avec l'organe de pression (142).

6. Système à vis ostéochirurgicale, selon l'une des revendications précédentes, **caractérisé par le fait que** l'outil rotatif (126), et l'organe de pression (142) de l'instrument de repositionnement (102), sont respectivement pourvus d'un passage d'outil destiné à un tournevis (158) dédié au vissage de la vis (152) de blocage à demeure dans le réceptacle frontal (18) de la vis pédiculaire (10).

7. Système à vis ostéochirurgicale, selon l'une des revendications précédentes, **caractérisé par le fait que** l'organe de pression (142) de l'instrument de repositionnement (102) présente un élément élastique, de préférence une bague élastique (150) destinée à retenir la vis (152) de blocage à demeure.

8. Système à vis ostéochirurgicale, selon l'une des revendications précédentes, **caractérisé par le fait que** la zone (44) de rupture par destination de la seconde aile de cloisonnement (32) de ladite vis ostéochirurgicale (10) inclut au moins une encoche de ladite seconde aile de cloisonnement (32).

9. Système à vis ostéochirurgicale, selon l'une des revendications précédentes, **caractérisé par le fait que** le segment (34) à filetage intérieur de la première aile de cloisonnement (30) de ladite vis ostéochirurgicale (10) s'étend, dans le sens axial, jusqu'à l'extrémité libre (42) de ladite première aile de cloisonnement (30) ; et **par le fait que** le segment (34) à filetage intérieur de la seconde aile de cloisonnement (32) s'étend jusqu'à la zone (44) de rupture par destination.

10. Système à vis ostéochirurgicale, selon l'une des revendications précédentes, **caractérisé par le fait que** le réceptacle frontal (18) est agencé, sur la tête (16) de la vis, avec faculté de déviation par rapport à l'axe longitudinal (22) de la tige filetée (12) de ladite vis ostéochirurgicale (10) et/ou est agencé, sur ladite tête (16) de la vis, à rotation libre autour de son axe longitudinal (20).

11. Système à vis ostéochirurgicale, selon l'une des revendications précédentes, **caractérisé par le fait que** le profil d'entraînement (48) est formé par une pièce intégrée (54) disposée dans un logement de montage (52) de la tête (16) de la vis, la pièce intégrée (54) étant assujettie en rotation à ladite tête (16) de la vis, avec orientation directionnelle autour de l'axe longitudinal (22) de la tige filetée (12) de ladite vis ostéochirurgicale (10), et ladite pièce intégrée (54) étant maintenue guidée sur le réceptacle frontal (18) et pouvant être déviée, conjointement à ce dernier, par rapport audit axe longitudinal de ladite tige filetée (12).

12. Système à vis ostéochirurgicale, selon l'une des revendications précédentes, **caractérisé par le fait que** le réceptacle frontal (18) est relié à la tête (16) de ladite vis par l'intermédiaire d'une bague élastique (58) préférentiellement fendue, ledit réceptacle frontal (18) étant intérieurement pourvu d'une rainure annulaire (62) et, à l'intérieur de ladite rainure annulaire (62), d'une saillie (64) ciblant une limitation radiale d'une expansion de ladite bague élastique (58).
